(19) Europäisches Patentamt
European Patent Office
Office européen des brevets

(11) Publication number: 0 167 936
A2

# (12) EUROPEAN PATENT APPLICATION

(21) Application number: 85107941.8

(51) Int. Cl.[4]: A 61 K 31/40

(22) Date of filing: 26.06.85

(30) Priority: 03.07.84 JP 136462/84

(43) Date of publication of application: 15.01.86 Bulletin 86/3

(84) Designated Contracting States: AT BE CH DE FR GB IT LI LU NL SE

(71) Applicant: MICROBIAL CHEMISTRY RESEARCH FOUNDATION
14-23, Kamiosaki 3 Chome Shinagawa-ku
Tokyo 141(JP)

(72) Inventor: Umezawa, Hamao, Prof.
23, Toyotama-kita 4-chome
Nerima-ku Tokyo(JP)

(72) Inventor: Aoyagi, Takaaki
3-6, Honkugenuma 3-chome
Fujisawa-city Kanagawa Prefecture(JP)

(72) Inventor: Takeuchi, Tomio
1-11, Higashigotanda 5-chome
Shinagawa-ku Tokyo(JP)

(72) Inventor: Hamada, Masa
26, Naito-cho 1-chome
Shinjuku-ku Tokyo(JP)

(72) Inventor: Ishizuka, Masaaki
17, Denenchofu-honcho 3-chome
Ohta-ku Tokyo(JP)

(74) Representative: Becker, Heinrich Karl Engelbert, Dr. et al,
HOECHST AKTIENGESELLSCHAFT Central Patent
Department P.O. Box 80 03 20
D-6230 Frankfurt am Main 80(DE)

## (54) Use of actinonin as an immunopotentiator.

(57) The present invention relates to the use of actinonin of the formula

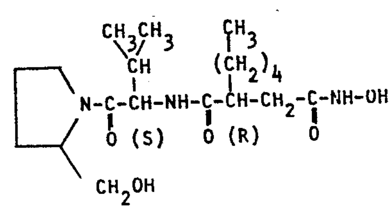

or one of its salts as immunopotentiator.

EP 0 167 936 A2

Croydon Printing Company Ltd

The present invention relates to the use of actinonin of the following formula

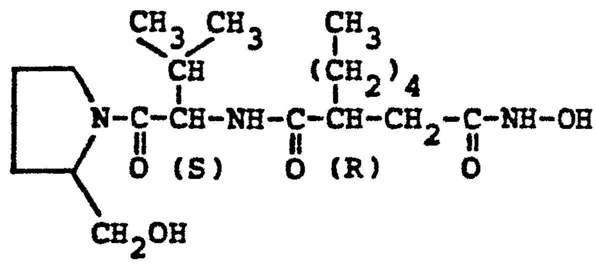

(hereinafter referred to as the present compound) or its salts as an immunopotentiator.

It has been found that inhibitors of the enzymes present on the surfaces of various cells have immunopotentiator action. The further search for substances enhancing cellular immunity, had the result that actinonin produced by MG848-hF6, a strain isolated by the Institute of Microbial Chemistry, has a marked immunoptentiating effect.

The compound actinonin is a compound known in the literature as having antibacterial activity (References: I.A.M. Symposia on Microbiology, No. 6, Chemistry of Microbial Product, 204-14 (1964) and U.S. Patent No. 3,240,787). Its physicochemical properties have also been known, but it remained unknown until now that the present compound has further, different pharmacological properties, particularly, the effect of potentiating cellar immunity.

The present compound is present in the cultured broth of the actinonin-producing strain MG848-hF6. This compound can be recovered in good yields by methods comprising filtrating the cultured broth, adsorbing the compound contained in the filtrated onto an adsorbent, and releasing it from the adsorbent.

The biological activities of the present compound will be described in the following:

A. The effect on cellular immunity in normal mice

(1) Method

The action of actinonin on cellular immunity was studied by way of delayed-type hypersensitivity (D.T.H.) that is produced by inoculating the footpad of the mouse with sheep red blood cells (SRBC) as an antigen (Reference: J. Exp. Med., 139, 1529 - 1539, 1974).

A suspension of $10^8$ SRBC in 0.05 ml of physiological saline was inoculated to a subdermal site of a footpad of 8-week-old female $CDF_1$ mice divided into groups of 6 mice each.

Simultaneously, an aqueous solution containing 5, 0.5, 0.05 or 0.005 mg/kg actinonin was administered intraperitoneally as a single dose. Four days after the administration, a suspension of $10^8$ SRBC in 0.05 ml of physiological saline was administered subcutaneously to the other footpad for secondary sensitization. Twenty-four hours later, the thickness of footpad was measured with a vernier caliper.

On the other hand, control animals were given subcutaneous injections of SRBC and physiological saline in accordance with the above procedure, but without being administered the test compound, and the thickness of the foodpad was measured in the same way. The increase in footpad thickness was evaluated to be 100 %.

The increase in foodpad thickness for animals treated with the test compound was compared with that for the control animals, thereby determining the cellular immunity-potentiating effect of the test compound.

(2) Results

| Test compound | Dose (mg/kg) | Increase in footpad thickness (X 0.1 mm) | Ratio to increase in footpad thickness of control (%) |
|---|---|---|---|
| Actinonin | 5 | 11.9 ± 1.44 | 138 * |
| | 0.5 | 11.4 ± 1.80 | 133 * |
| | 0.05 | 10.4 ± 1.40 | 121 |
| | 0.005 | 9.2 ± 1.15 | 107 |
| Bestatin | 0.5 | 12.8 ± 1.47 | 149 * |
| Control | | 8.6 ± 1.78 | 100 |

* Significantly different from the increase in footpad thickness of the control ($P < 0.05$)

As disclosed above, actinonin at doses of 5 and 0.5 mg/kg increased the footpad of mice singificantly, as seen from the values 138 and 133 %, respectively, although its effect was slightly lower than the reference drug bestatin given at a dose of 0.5 mg/kg.

B. The effect of cellular immunity by sensitization with picryl chloride in carcinomatous mice

(1) Method

$10^6$ cells of Ascites Sarcoma 180 were implanted intraperitoneally into 12-week-old female $CDF_1$ mice divided into groups of 6 mice each. The day of implantation was designated as Day 0. On Day 1, the left ear was sensitized with 30 µl of a 1 % ethanol solution of picryl chloride absorbed to an absorbent cotton cut to a size of 10 mm x 5 mm x 1 mm.

Then, 5.0, 0.5, 0.05 or 0.005 mg/kg of actinonin dissolved in physiological saline was orally administered once

daily from Day 1 to Day 8, with the exception of Days 5 and 6 when the test compound was not administered. During the period, the control group was given physiological saline only. On Day 8 the right ear was sensitized with 20 µl of a 1 % olive oil solution of picryl chloride absorbed to an absorbent cotton cut to a size of 10 mm x 5 mm x 1 mm. Twenty-four hours later, the thickness of the ear was measured with a dial gauge.

On the other hand, the control animals were given picryl chloride and physiological saline in accordance with the same procedure, but without being treated with the test compound, and the thickness of the ear was measured in the same way. The increase in this thickness for the control group as evaluated to be 100 %.

The increase in the thickness of the ear for the group treated with the test compound was compared with that for the control group, thereby to determine the cellular immunity-potentiating effect of the test compound.

(2) Results

| Test compound | Dose (mg/kg) | Increase in footpad thickness ($x 10^{-3}$ cm) | Ratio to increase in footpad thickness of control (%) |
|---|---|---|---|
| Actinonin | 5 | 4.60 ± 1.19 | 94.8 |
| | 0.5 | 7.08 ± 1.66 * | 146.0 |
| | 0.05 | 6.80 ± 0.84 * | 140.2 |
| | 0.005 | 4.70 ± 0.67 | 96.9 |
| Bestatin | 0.5 | 6.75 ± 1.51 | 139.2 |
| Control | | 4.85 ± 0.58 | 100 |

* $P < 0.05$

As noted above, actinonin exhibits a significant action of potentiating cellular immunity that is comparable to the action of the reference drug bestatin.

The above results put together demonstrate actinonin to potentiate cellular immunity not only in normal animals but it immunocompromised animals with carcinoma.

Acute toxicity studies in mice showed the present compound to cause no deaths at a dose of 400 mg/kg. Thus, the present compound is considered to be a safe substance.

As described above, actinonin in accordance with this invention enhances immunity when administered alone, and exhibits a carcinostatic effect by a host-mediated mechanism. Accordingly, the compound of this invention is useful as an immunomodulator and an antitumor immunopotentiator, or as an adjuvant for chemotherapeutic agents for use in the treatment of various types of cancer.

Such drugs containing actinonin as the active ingredient can be prepared by mixing actinonin or its pharmaceutically acceptable salts with carriers which are in customary use. Various chemotherapeutics may be further incorporated into these preparations.

The compound of this invention and pharmaceuticals comprising it may be administered as oral preparations, injections or rectal suppositories. Lyophilized injections can be prepared by adding pH regulators, buffers, stabilizers, vehicles, etc. to the present compounds the active ingredient, adn freeze-drying the mixtures by customary methods. Hypodermic, intramuscular and intravenous injections can be prepared by blending the present compound as the active ingredient with pH regulators, buffers, stabilizer, isotonizers, local anesthetics, etc. and processing the blends by customary methods.

Oral solids, such as tablets, coated tablets, granules, powders and capsules, can be prepared by adding vehicles, and if desired, binders, disintegrators, lubricants, colorants, flavoring agents, and odorizers, to the present compound as the active ingredient, and then processing the mixtures in the customary manner.

Oral liquids, such as syrups, and dry syrups, can be prepared by adding flavoring agents, buffers, stabilizers, odorizers, etc. to the present compound as the active ingredient, and processing the mixtures in the customary manner.

Rectal suppositories can be prepared by adding vehicles, and if desired, surfactants, to the present compound as the active ingredients and processing the mixtures by customary methods.

The dose of actinonin vary with symptoms. In adults, the recommended dose is 1 to 200 mg of actinonin given once daily. When actinonin is concomitantly used with other carcinostatic chemotherapeutic agents or immunopotentiators, it is recommendable that actinonin within said dose range be combined with a carcinostatic chemotherapeutic or immunopotentiator in the usual dose.

The production of the present compound will be described by way of the following Referential Example.

<u>Referential Example</u>

The culture broth of actinonin-producing strain MG848-hF6 that has been cultivated by customary method is filtered. The filtrate is passed through a column packed with 1/10 volume of $^{R}$Amberlite XAD-4 to adsorb the present compound to the column. The $^{R}$Amberlite XAD-4 in the column is washed with water, and eluted with an 80 % aqueous solution of methanol.

The eluate is concentrated to dryness under reduced pressure to obtain crude powder I. The crude powder I is chromatographed on silanized silica gel and eluted with a gradient of acetonitrile ranging from 0 to 80 % in a buffer (pH 4.9) consisting of 1 % citric acid and 2 % potassium acetate, thereby to obtain fractions containing the present compound. The active fractions are concentrated under reduced pressure to a 1/10 volume, and the concentrate is applied to a column of $^{R}$Amberlite XAD-4 for desalting. The $^{R}$Amberlite XAD-4 in the column is washed with water, and

eluted with an 80 % aqueous solution of methanol. The eluate is concentrated to dryness under reduced pressure to obtain crude powder II. The crude powder II is chromatographed on silica gel, and eluted with chloroform-methanol (95 : 5) to obtain fractions containing the present compound. The active fractions are solidified under reduced pressure to isolate the present compound. Recrystallization of the isolated compound from benzene-methanol gives pure product.

Actinonin in the cultivation and purification steps was traced based on aminopeptidase M inhibitory activity determined by the following method.

0.5 ml of 0.1 M Tris-HCl buffer (pH 7.0) and 0.2 ml of a solution containing the test compound were added to 0.25 ml of a solution of 0.002 M leucine-ß-naphthylamide (Bachem Feinchemikalien AG). The mixture was heated for 3 minutes at 37°C, and 50 µl of a solution of aminopeptidase M (Boehringer Mannheim) from swine kidney was added.

After the system was reacted for 30 minutes at 37°C, 1 ml of 1.0 M acetate buffer (pH 4.2) containing Fast Garnet GBC (orthoaminoazotoluene diazonium salt) at a concentration of 1 mg/ml and the surfactant Tween 20 at a concentration of 10 % was added to the reaction mixture. After being allowed to stand for 15 minutes at room temperature, the mixture was measured for absorbance at 525 nm, which was designated as absorbance (a).

Separately, the blank containing no actinonin and using only the buffers was measured for absorbance, which was designated as absorbance (b).

The aminopeptidase M inhibition rate was calculated form the equation $[(b-a)/b] \times 100$ (%). Actinonin at a concentration of 0.4 µg/ml produced 50 % inhibition ($IC_{50}$) of aminopeptidase M activity.

Claims

1. Use of actinonin of the formula

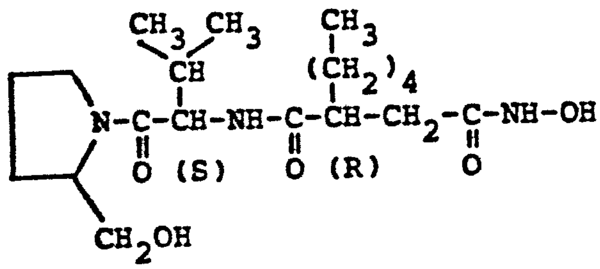

or one of its salts for the manufacture of a medicament having immunopotentiating activity.

2. A pharmaceutical composition having immunomodulating activity which comprises actinonin of the formula

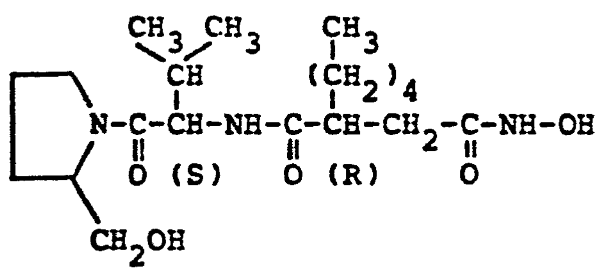

or one of its salts as the active ingredient in association with a pharmaceutically suitable carrier and/or auxiliary.

Claim for Austria:

Use of actinonin of the formula

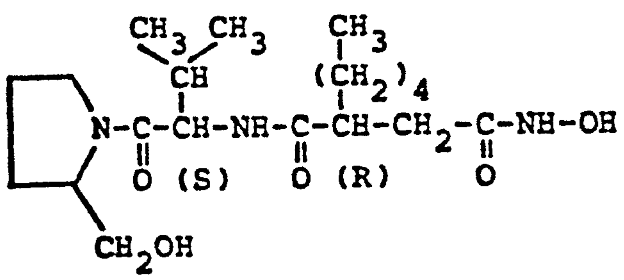

or one of its salts for the manufacture of a medicament having immunopotentiating activity.